(19) Europäisches Patentamt
European Patent Office
Office européen des brevets

(11) **EP 4 258 206 B1**

(12) **EUROPEAN PATENT SPECIFICATION**

(45) Date of publication and mention
of the grant of the patent:
**25.02.2026  Bulletin 2026/09**

(21) Application number: **22167134.0**

(22) Date of filing: **07.04.2022**

(51) International Patent Classification (IPC):
***G06T 7/00*** *(2017.01)*      ***G06T 7/11*** *(2017.01)*

(52) Cooperative Patent Classification (CPC):
**G06T 7/0012; G06T 7/11;** G06T 2207/10081;
G06T 2207/10088; G06T 2207/20081;
G06T 2207/20084; G06T 2207/30084;
G06T 2207/30096

(54) **METHODS AND SYSTEMS FOR CLASSIFYING A MALIGNANCY RISK OF A KIDNEY AND TRAINING THEREOF**

VERFAHREN UND SYSTEME ZUM KLASSIFIZIEREN EINES MALIGNITÄTSRISIKOS EINER NIERE UND TRAINING DAVON

MÉTHODES ET SYSTÈMES POUR CLASSER UN RISQUE DE MALIGNITÉ D'UN REIN ET ENTRAÎNEMENT CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**11.10.2023  Bulletin 2023/41**

(73) Proprietor: **Siemens Healthineers AG**
**91301 Forchheim (DE)**

(72) Inventors:
• **GRBIC, Sasa**
**Plainsboro, 08536 (US)**
• **GEIGER, Bernhard**
**Cranbury, 08512 (US)**
• **HÖLZER, Philipp**
**91088 Bubenreuth (DE)**

(74) Representative: **Kraus & Lederer PartGmbB**
**Thomas-Wimmer-Ring 15**
**80539 München (DE)**

(56) References cited:
• **DAN ZHAO ET AL: "Deep Learning in Computer-Aided Diagnosis and Treatment of Tumors: A Survey", ARXIV.ORG, CORNELL UNIVERSITY LIBRARY, 201 OLIN LIBRARY CORNELL UNIVERSITY ITHACA, NY 14853, 2 November 2020 (2020-11-02), XP081805486**
• **JANG HYUN-JONG ET AL: "Applications of deep learning for the analysis of medical data", ARCHIVES OF PHARMACAL RESEARCH, NATL. FISHERIES UNIVERSITY , PUSAN, KR, vol. 42, no. 6, 28 May 2019 (2019-05-28), pages 492 - 504, XP037240316, ISSN: 0253-6269, [retrieved on 20190528], DOI: 10.1007/S12272-019-01162-9**

**Description**

TECHNICAL FIELD

Examples of the invention generally relate to methods and systems for classifying a malignancy risk of a kidney. Further examples of the invention relate to methods and systems for training a machine learning algorithm to classify a malignancy risk of a kidney.

BACKGROUND

Determining the malignancy risk for renal lesions is essential for patient management. Especially the distinction of cysts, which are present in more than 50 % of people above 50 years, and cancerous lesions is important. Imaging, especially CT and MRI, plays a crucial part in determining the malignancy risk for complex lesions. However, often the criteria defined are ambiguous and often not consistent between physicians. This leads to suboptimal outcomes for patients.

For some of the lesions ultrasound imaging can be used to distinguish cysts. However, this approach only works for "simple cysts" which only have clear fluid inside and are harmless. More complex cysts need to examine with more advanced imaging such as CT and MRI.

There are several guidelines to examine, and report suspected lesions such as the Bosniak criteria. For example, the Bosniak criteria defines a cystic renal mass as a mass that, based on subjective visual inspection, is composed of less than approximately 25 % enhancing components. Therefore, masses with approximately 25% or more enhancing components are considered solid and not classifiable by the Bosniak classification.

The distinction between cystic and solid renal masses is important. Solid masses behave more aggressively and have a higher propensity for local recurrence and metastatic disease than cystic masses. Therefore, their management and prognosis differ. As a corollary, it is important not to mistake a necrotic RCC for a cystic renal cell carcinoma (and vice versa), as the former is aggressive and the latter is typically indolent.

However, the guidelines are not very specific which can lead to large user variability and disagreements between individual readers. Thus, the risk assessment and ultimately the patient management can be quite different depending on who is examining the imaging data. Additionally, as the guidelines are based on "simple" appearances that can be interpreted by physicians the performance of such a classification is limited as much more sophisticated imaging features are not incorporated. And this sometimes leads to sub-optimal patient outcomes.

Dan Zhao et al., "Deep Learning in Computer-Aided Diagnosis and Treatment of Tumors: A Survey", ARXIV.ORG, Cornell University Library, Ithaca, NY 14853, 2 November 2020, pages 1 - 17 discloses a survey on computer-aided tumour diagnosis using images and pathological data from CT or MR images using e.g. CNNs like a Fully Convolutional Network with an encoder-decoder model. Applications can be tumour segmentation in a kidney and pathological diagnosis.

Hyun-Jong Jang et al., "Applications of deep learning for the analysis of medical data", Archives of Pharmacal Research, Natl. Fisheries University, Pusan, KR, vol. 42, no. 6, 28 May 2019, pages 492 - 504, ISSN: 0253-6269, DOI: 10.1007/S12272-019-01162-9 discloses a trained CNN classification of lesions, such as in MR images of the kidney.

SUMMARY

Thus, a problem of the present invention may be stated as how to improve the classification of malignancy risks of a kidney, in particular a human kidney. The invention is defined in the independent claims; advantageous embodiments are defined in the dependent claims.

A first aspect of the invention refers to a computer-implemented method for classifying a malignancy risk of a kidney, in particular a human kidney. The method therefore comprises the steps of providing imaging data of an anatomy of a subject patient, wherein the imaging data comprises at least partially a representation of a kidney of the subject patient, using a first neural network to segment at least one region of the kidney representation which is based on the imaging data and using a second neural network to detect one or more suspected lesions of the segmented kidney representation. Furthermore, the step of classifying the detected suspected lesion with a malignancy risk using a third neural network is performed, wherein the third neural network is a deep profiler.

By using a deep profiler to determine the malignancy risk of suspected renal lesions the method can extract complex features from imaging data to create a more accurate risk score. In particular, classifying the malignancy risk improves the patient management for patients with suspected renal lesions.

A second aspect of the invention refers to a system for classifying a malignancy risk scoring of a kidney, in particular a human kidney. The system comprises an interface which is configured to provide imaging data of an anatomy of a subject patient, wherein the imaging data comprises at least partially a representation of a kidney of the subject patient.

Furthermore, the system comprises at least a first analyzing unit which is configured to use a first neural network to segment at least one region of the kidney representation which is based on the imaging data, a second analyzing unit which is configured to use a second neural network to detect one or more suspected lesions of the segmented kidney representation, and a deep profiler which is configured to classify the detected suspected lesion with a malignancy risk.

Deploying a system according the second aspect of the invention may provide efficient and accurate assessment of the malignancy risk. Based on that, such a system can be deployed anywhere and thus the system deployment is not limited to any certain geographical areas where a specific kidney expert is practicing. In addition, this system can be scaled easily, which means that it may not be limited to a low number of classifications per day as it may be the case when kidney imaging data are classified manually.

In one example according the first and/or second aspect, the third neural network is configured to classify the malignancy risk based on imaging data and non-imaging data, wherein the non-imaging data comprises at least histopathologic data. Histopathologic data can pertain to a result of a histopathologic assessment; a microscopic image of a tissue sample can be inspected for tumor-infested tissue, e.g., using fluorescence microscopy.

Non-imaging data may additionally support the classification of malignancy risks since imaging data itself may be incomplete or biased by not accurate enough image recording. In particular, histopathologic data can improve the malignancy risk classification to the extent that false-positive samples can be avoided up to 90 %, preferably up to 95 % and more preferably up to 99 %. Thus, an unnecessary surgical intervention can be avoided for a subject patient. This may reduce healthcare costs and improved patient satisfaction due to reduction in follow-up exams. Furthermore, this may reduce the number of missed malignant lesions and thus may improve the patient survival probability.

In one example according the first and/or second aspect, the deep profiler comprises an encoder for extracting imaging features. Utilizing an encoder may support the deep profiler to build at least one task-specific fingerprint. An encoder can contract spatial features in one or more steps to determine a latent feature vector.

In one example according the first and/or second aspect, the encoder is designed as convolutional neural network (CNN), in particular a three-dimensional CNN. Applying a CNN may reduce or even minimize the cost of computing since CNNs make us of local spacial coherence that provides same weight to some of the edges. This is in particular useful when GPU resources are limited which may be often the case in medical environments where standard computers or only handheld devices like tablet PCs or smartphones are used for classification. In addition, CNNs may reduce the size of memory which is needed for execution due to a reduced number of parameters. Compared to a simple deep neural network, a reduced number of hidden layers and nodes may reduce the allocated memory needed for execution.

Moreover, deploying CNNs may help the method or system for classifying a malignancy risk since minor or major changes in the output data may be identified more accurately and retain the reliability of the model. This is based on the fact that convolutions are equivariant to many data transformation operations which may help to identify how a particular change in input will affect the output. Three-dimensional CNNs may benefit even more from the above identified advantages.

In one example the deep profiler comprises a decoder for estimating at least one malignancy risk indicator. A decoder may expand spatial features in one or more steps, based on a latent feature vector. By estimating one or more approximated imaging data, the decoder can generate synthetic imaging data which can be compared to real-world imaging data again. Any further information like deviations from real-world imaging data and/or deviations from ideal imaging data can be used for further classifications again.

In one example according the first and/or second aspect, the deep profiler comprises a task-specific network for generating at least one image signature for classifying at least one malignancy risk. Using one or more image signatures may decrease the false-positive rate of detected lesions of kidneys. Furthermore, comparing imaging data of subject patients with image signatures may be more efficient since specific parameters which may be characterizing for a high or low malignancy risk can be emphasized in image signatures.

In one example according the first aspect, the method comprises the additional method step: using a fourth neural network to detect anatomical landmarks based on the provided imaging data. This additional step may support the classification since the provided imaging data may be prefiltered. Thus, not-relevant parts of the anatomy of a subject patient, which are incorporated in the imaging data, can be reduced or even minimized.

In one example according the first and/or second aspect, the fourth neural network is designed as convolutional neural network for image feature extraction. CNNs for image feature extraction may be more beneficial compared to other machine learning algorithms since CNNs are more independent to geometrical transformations like scaling or rotations. Thus, the image feature extraction may be improved.

In one example according the first and/or second aspect, the fourth neural network additionally uses at least one universal non-linear function approximator. Compared to mainly linear function approximators, non-linear function approximators are beneficial due to the fact that they may decrease the error rate compared to the number of parameters in the approximant. In addition, the number of parameters usually correlates with computational efforts. Thus, non-linear functions may help to reduce computational efforts, too. It is shown that in many settings the rate of nonlinear approximation can be characterized by certain smoothness conditions which are significantly weaker than required in the linear theory.

In one example according the first and/or second aspect, the first neural network is designed as convolutional encoder-decoder architecture. This architecture may reduce or even skip connections. Furthermore, a convolutional encoder-decoder setup may support a deep supervision scheme within a framework to improve its structure design compared to a U-Net or deep supervised networks. This is in particular beneficial for 3D volumetric datasets which may be the case for the present invention.

In one example according the first and/or second aspect, the first neural network is designed as multi-level feature concatenation and deep supervision architecture. Therefore, bridges may be built directly from the encoder layers to the decoder layers. These bridges may pass information from the encoder forward and then concatenate it with decoder feature layers. Based on that, the first neural network may benefit from local and global contextual information and may achieve improved boundary detection and segmentation results.

In one example the step of detecting one or more suspected lesions is based on a fully convolutional one-stage object detection (FCOS). The FCOS approach may be anchor box free and proposal free. Additionally, by eliminating the predefined set of anchor boxes, FCOS may avoid complicated computation related to anchor boxes such as calculating overlapping. Furthermore, several or even all hyper-parameters related to anchor boxes may be avoided which may be sensitive to a specific detection performance. Moreover, FCOS may surpass previous one-stage detectors with the advantage of being much simpler and more flexible regarding its detection framework which may result in an improved detection accuracy.

In one example according the first and/or second aspect, the provided imaging data is based on computer tomography (CT) and/or magnet resonance imaging (MRI). Since image capturing based on CT or MRI is widely applied around the world, a minimum level of quality and accuracy may be guaranteed as inputted imaging data. Furthermore, CT and/or MRI imaging data may be captured in a three-dimensional manner, thus additionally improving and enriching the available imaging data. Since CT and MRI are standard image capturing methods it may be guaranteed that subject patient can be examined with at least one of CT and/or MRI in most of the time.

In one example according the first and/or second aspect, the imaging data and/or non-imaging data comprises context data relating to data recording information. It may be necessary to access data recording information additionally when e.g. chromaticity parameters of the imaging data differ from ideal data or data which were used for training the method or system. Moreover, imaging data may differ based on the image capturing devices from different manufacturers and thus, it may be necessary to align the different captured imaging data accordingly.

In one example according the first and/or second aspect, the context data comprises at least one out of following: data recording device information, contrast configuration information, brightness configuration information, recording direction information, total recording time information, projection type information (e.g. CT: average intensity projection (AIP), maximum intensity projection (MIP) or Minimum intensity projection MinIP)), date and/or time of recording information. Thus, any deviations from other imaging data can be standardized and thus corrected based on the context data.

In one example according the first and/or second aspect, the non-imaging data of the subject patient comprises at least one out of following: age, weight, size, health condition information, gender, nutritional practice. Based on these data which refer to the subject patient any deviations from at least two different imaging data can be at least partly aligned or corrected to improve the classification additionally.

In one example according the first and/or second aspect, the provided imaging data comprises at least partially a 3D illustration of the anatomy of the subject patient. This may improve the classification of a malignancy risk since different layers of a detected kidney lesion can be analyzed individually and thus the scoring may be more accurate.

In one example according the first aspect, the method comprises the additional step: converting the imaging data from at least a partially 3D illustration of the anatomy of the subject patient to a 2D illustration of the anatomy of the subject patient. This step may be necessary whenever limited computational resources are available. When converting the 3D illustration to a 2D illustration, the volume of imaging data can be reduced and thus, less memory space and computational effort for analyzing the data is needed.

A third aspect refers to a computer-implemented method for training a machine learning algorithm to classify a malignancy risk of a kidney, in particular a human kidney. The method comprises the steps of training a first neural network with first training data including imaging data of an anatomy of at least one subject patient, wherein the imaging data comprises at least partially a representation of one or more kidneys, training a second neural network with second training data including one or more detected lesions of one or more segmented kidney representations and training a third neural network with third training data of one or more lesions classified with a malignancy risk.

A fourth aspect of the invention refers to a system for training a machine learning algorithm to classify a malignancy risk of a kidney, in particular a human kidney, comprising a first analysis unit which is configured to train a first neural network with first training data including imaging data of an anatomy of at least one subject patient, wherein the imaging data comprises at least partially a representation of one or more kidneys, a second analysis unit which is configured to train a second neural network with second training data including one or more detected lesions of one or more segmented kidney representations; and a third analysis unit which is configured to train a third neural network with third training data of one or more lesions classified with a malignancy risk.

A method or system for training a machine learning algorithm to classify a malignancy risk of a kidney may be beneficial due to the fact that it may reduce the error-rate in patient-management of patients which are suspected with renal lesions. In particular, it may increase inconsistency of documented exam information and may decrease the likelihood of user/institution bias. The reason therefore may be seen that a machine learning algorithm according to the invention may be much more accurate compared to a manual training of a single expert or a group of experts. In particular, inexperienced experts like radiographers may not have the experience compared to a trained machine learning algorithm. Furthermore, by training a machine learning algorithm the classification algorithm may be much faster educated compared to a human expert. In addition, it is nearly impossible for a human expert to analyze and learn his/her manual classification accuracy with such a high number of classified examples compared to a computer-based training.

In one example according to the third and/or fourth aspect, the third training data are based on imaging data and non-imaging data, wherein the non-imaging data comprises at least histopathologic data.

Based on histopathologic data specific information which is inherent for any malignancy risk classification of kidneys can be added to the machine learning algorithm which may result in much higher accurate classifications.

In one example according to the third and/or fourth aspect, at least one ground-truth label of the deep profiler is determined based on histopathologic data. Thus, the method or system for training may be more precisely if at least one ground-truth label refers at least partly to histopathologic data.

In one example according to the third aspect, the method comprises the following additional step of training a fourth neural network with fourth training data using deep reinforcement learning based on training data related to detected anatomical landmarks, in particular landmarks relating to one or more kidney representations. Based on this, the first, second and/or third training data can be improved with more accurate and relevant data. In particular, irrelevant imaging data information can be reduced or even removed. Thus, the quality of the training data, in particular imaging data, may be improved.

In one example according to the third and/or fourth aspect, an adversarial network is utilized for training of the first neural network to discriminate the output from ground truth. Therefore, the first neural network pushes its output towards the distribution of ground truth, sot that it may enhance its performance by refining its output. By doing so, a higher computing efficiency may be achieved since the discrimination does not need to be executed at any inference.

In one example according to the third and/or fourth aspect, at least two different training data fragments are based on the same subject patient. In one example according to the third and/or fourth aspect, the two different training data fragments are indicated as training data of the same subject patient. In one example according to the third and/or fourth aspect, the non-imaging data comprise at least one indicator which is configured to serve for patient follow-up diagnosis. Thus, it can be achieved that the training method or system is able to track the malignancy risk of a specific subject patient, in particular over time.

A fifth aspect of the invention relates to a computer program comprising instructions which, when the program is executed by a computer, cause the computer to carry out at least one of the examples of the method according the first aspect or according the third aspect of the invention.

A sixth aspect of the invention refers to a machine-readable medium having stored thereon a set of instructions, which if performed by one or more processors, cause the one or more processors to carry out at least one of the examples of the method according the first aspect or according the third aspect of the invention.

BRIEF DESCRIPTION OF THE DRAWINGS

Further details and advantages of the present invention can be taken from the following description of preferred examples in conjunction with the drawings, in which:

FIG. 1 is a schematic drawing depicting a computer-implemented method for classifying a malignancy risk of a kidney;

FIG. 2 is a schematic drawing depicting a system for classifying a malignancy risk scoring of a kidney;

FIG. 3 is a schematic drawing depicting a computer-implemented method for training a machine learning algorithm to classify a malignancy risk of a kidney; and

FIG. 4 is a schematic drawing depicting a system for training a machine learning algorithm to classify a malignancy risk of a kidney.

DETAILED DESCRIPTION OF THE EXAMPLE EXAMPLES

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Various examples will now be described more fully with reference to the accompanying drawings in which only some examples are shown. Specific structural and functional details disclosed herein are merely representative for purposes of describing examples. Examples, however, may be embodied in various different forms, and should not be construed as being limited to only the illustrated examples. Rather, the illustrated examples are provided as examples so that this disclosure will be thorough and complete, and will fully convey the concepts of this disclosure to those skilled in the art. Accordingly, known processes, elements, and techniques, may not be described with respect to some examples. Unless otherwise noted, like reference characters denote like elements throughout the attached drawings and written description, and thus descriptions will not be repeated. The present invention, however, may be embodied in many alternate forms and should not be construed as limited to only the examples set forth herein.

It will be understood that, although the terms first, second, etc. may be used herein to describe various elements, components, regions, layers, and/or sections, these elements, components, regions, layers, and/or sections, should not be limited by these terms. These terms are only used to distinguish one element from another. For example, a first element could be termed a second element, and, similarly, a second element could be termed a first element, without departing from the scope of example examples of the present invention. As used herein, the term "and/or," includes any and all combinations of one or more of the associated listed items. The phrase "at least one of" has the same meaning as "and/or".

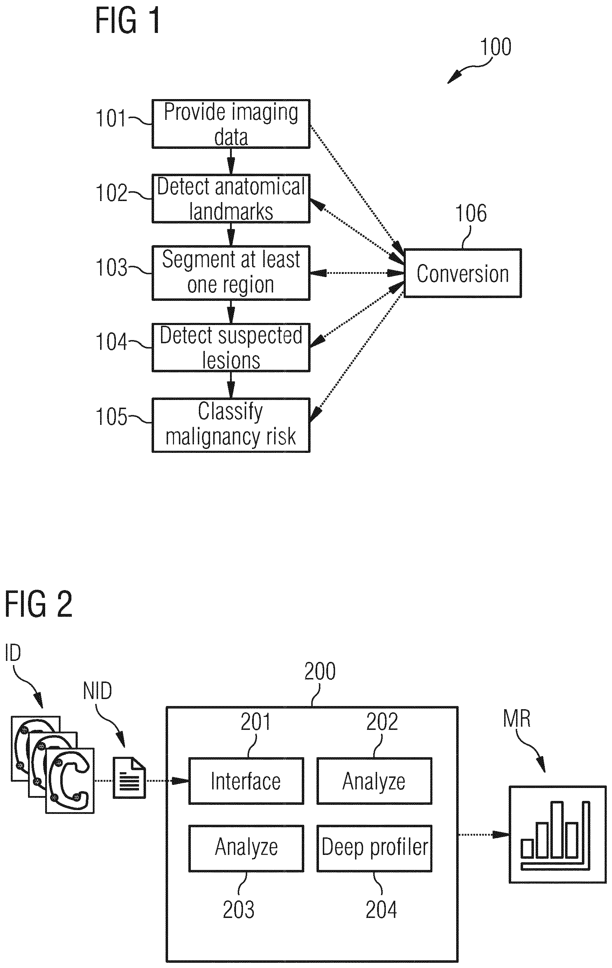

FIG. 1 is a schematic drawing depicting a computer-implemented method 100 for classifying a malignancy risk MR of a kidney.

In a first method step 101 imaging data of an anatomy of a subject patient is provided. The imaging data ID comprises at least partially a representation of a kidney of the subject patient. The imaging data ID may be based on image capturing via computer tomography (CT) and/or magnet resonance imaging (MRI) .

Thus, the provided imaging data ID may comprise at least partially a 3D illustration of the anatomy of the subject patient. In particular, the 3D illustration is layered, which means that at least two layers can be extracted from the imaging data ID defining at least two different depths of the captured anatomy.

In a second method step 102 a fourth neural network is used to detect anatomical landmarks based on the provided imaging data ID. This step is in particular important since the method may require a precise, automatic detection of anatomical structures, preferably of at least a kidney, to initialize and constrain mathematical models for volumetric organ segmentation. As such, enabling accurate and efficient anatomical landmark detection can support the method 100 for a more effective and streamlined image reading.

Therefore, the fourth neural network may be designed as convolutional neural network for image feature extraction. Furthermore, the fourth neural network may additionally use at least one universal non-linear function approximator.

The network may be parametrized by $\Theta = [W, b]$, where W denotes the inter-neural connection weights organized as (multichannel) filter kernels, and b defines the set of neuron bias values.

Convolutional layers may exploit local spatial correlations of image voxels to learn translation-invariant convolutional kernels, which capture discriminative image features. It may be considered a multi-channel signal representation $M_k$ in layer k, i.e., a channel-wise concatenation of signal representations $M_{k,c}$ with $c \in \mathbb{N}$ . One can generate a signal representation in layer k + 1 as: $M_{k+1,l} = \phi(M_k * w_{k,l} + b_{k,l})$ where $W_{k,l} \in W$ may represent a convolutional kernel with the same number of channels as $M_k$, the value $b_{k,l} \in b$ may represent the bias, l denotes the channel index, and * denotes a convolution operation.

The function $\phi$ may represent the nonlinear activation function, which is applied pointwise. Rectified linear unit (ReLU) activations may be used. The final network layers may be typically fully-connected. In a supervised regression setup, given training data $D = [(X_{1,y1}), ..., (X_{N,YN})]$, i.e., N independent pairs of volumetric image observations with value assignments, one may define the network response function as $R(\cdot; \theta)$, and use Maximum Likelihood Estimation to estimate the optimal network parameters (L denotes the likelihood):

$$\hat{\theta} = argmax\, L(\theta; D) = argmin \sum_{i=1}^{N} (R(X_i; \theta) - y_i)^2$$

This optimization problem may be solved with stochastic gradient descent (SGD) combined with the back-propagation algorithm to compute the network gradients.

It may be beneficial to reformulate the anatomy detection as a cognitive learning task for an artificial agent. Given a volumetric image I : $\mathbb{Z}^3 \rightarrow \mathbb{R}$ and the location of an anatomical structure of interest $\vec{p}_{GT} \in \mathbb{R}^3$ within I, the task may to learn a navigation strategy to $\vec{p}_{GT}$ in image space, i.e., a voxel grid of the imaging data ID. In other words, to seek voxel-based navigation trajectories from any arbitrary starting point $\vec{p}_0$ to $\vec{p}_k$ within image I, with the property that $\|\vec{p}_k - \vec{p}_{GT}\|$ is minimal. With reinforcement learning this problem may be modelled using a Markov Decision Process (MDP) M := (S, A, T, R, γ), where:

S may represent a finite set of states, $s_t \in S$ being the state of the agent at time t. To encode the location of the agent in the imaged volumetric space at time t, it may be defined $s_t = I(\vec{p}_k)$, which may denote an axis-aligned box of image intensities extracted from I and centered at the voxel-position $\vec{p}_t$ in image space.

A may represent a finite set of actions allowing the agent to interact with the environment defined by I, where $a_t \in A$ is the action the agent may perform at time t. A discrete voxel-wise navigation model may be used allowing the agent to move from any voxel position $\vec{p}_t$ to an adjacent voxel position $\vec{p}_{t+1}$ in image space.

$T : S \, x \, A \, x \, S \rightarrow [0; 1]$ may be a stochastic transition function, where $T_{s,a}^{s'}$ may describe the probability of arriving in state s', after performing action a in state s. $R : S \, x \, A \, x \, S \rightarrow \mathbb{R}$ may be a scalar reward function, which drives the behavior of the agent, where $R_{s,a}^{s'} \in \mathbb{R}$ may denote the expected reward after a state transition. For a state transition s → s' at time t from $\vec{p}_t \rightarrow \vec{p}_{t+1}$, we define $R_{s,a}^{s'} = ||\vec{p}_t - \vec{p}_{GT}||_2^2 - ||\vec{p}_{t+1} - \vec{p}_{GT}||_2^2$. This may represent a distance-based feedback, which is positive if the agent gets closer to the target structure and negative otherwise. γ may be the discount factor controlling the importance of future versus immediate rewards.

Furthermore, an optimal action-value function Q*(..,..) may be defined which encodes the maximum expected future discounted reward when starting in state s, performing action a, and acting optimally thereafter:
Q*(s, a) = maxE[R_t|s_t = s, a_t = a, π], where π may be an action policy, in other words a probability distribution over actions in any given state. An important relation satisfied by the optimal action-value function Q* may be the Bellman optimality equation, which represents following recursive formulation:

$$Q^*(s, a) = \sum T_{s,a}^{s'} \left( R_{s,a}^{s'} + \gamma maxQ^*(s', a') \right) = E_{s'}(r + \gamma maxQ^*(s', a'))$$, where s' defines a possible state visited after s, a' the corresponding action and $r = R_{s,a}^{s'}$ represents a compact notation for the current, immediate reward. In one example, this approach may be amended by deploying a deep Q-network (DQN) which is used as a non-linear approximator for the optimal action-value function. Accordingly, a deep Q-network can be trained in an RL setup using an iterative approach to minimize the mean squared error based on the Bellman optimality equation. At any training-iteration i, it may be possible to approximate an optimal expected target value for the action-value function using a set of reference parameters based on a previous training iteration i' < i.

Learning the action-value function Q* may enable the agent to effectively search for objects in the image, as opposed to scanning the volumetric space exhaustively. This learning process may be based on an adequate exploration of the environment, which we ensure through an off-policy ε - *greedy* approach.

The variable ε ε [0,1] controls the randomness in the exploration. This means that during training, actions are selected either uniformly at random with probability ε, or deterministically using the current policy with probability 1 - ε. Another important strategy to ensure the training stability may be the decorrelation of the training samples using the concept of experience replay. During training, the agent maintains an active memory of episodic trajectories M = [T1; T2; ...], which is constantly expanded and uniformly sampled to estimate the learning gradient.

To further accelerate the training, it may be possible to use an adaptive episode length. By gradually reducing the episode length during training using linear decay, the space exploration by sampling increasing numbers of trajectories that are stored in the active memory may be improved.

In a third method step 103 a first neural network is used to segment at least one region of the kidney representation which is based on the imaging data ID. Therefore, the first neural network may be designed as convolutional encoder-decoder architecture. Additionally, the first neural network may be designed as multi-level feature concatenation and deep supervision architecture. Compared to non-learning-based approaches like statistical distribution of the intensity, including atlas-based, active shape model (ASM-)based, level-set based or graph-cut-based methods, learning-based approaches may be more beneficial for better segmentation.

Fully convolutional networks (FCN) with deep supervision may be used, which can perform end-to-end learning and inference. The output of FCN may be refined with a fully connected conditional random field (CRF) approach. Furthermore, cascaded FCNs followed by CRF refinement may be applied.

However, also Generative Adversarial Networks (GAN) may be a powerful framework for this task. The GAN may comprise at least a generator and a discriminator. The generator tries to produce the output that is close to the real samples, while the discriminator attempts to distinguish between real and generated samples.

An advanced approach may be an adversarial image-to-image network (DI2IN-AN), wherein a deep image-to-image network (DI2IN) may serve as a generator to produce a liver segmentation. It may employ a convolutional encoder-decoder architecture combined with multi-level feature concatenation and deep supervision. The network may try to optimize a conventional multi-class cross-entropy loss together with an adversarial term that aims to distinguish between the output of DI2IN and ground truth.

Ideally, the discriminator pushes the generator's output towards the distribution of ground truth, so that it may have the potential to enhance generator's performance by refining its output. Since the discriminator is usually a CNN which takes the joint configuration of many input variables, it may embed the higher-order potentials into the network (the geometric difference between prediction and ground truth is represented by the trainable network model instead of heuristic hints). The proposed implementation also achieves higher computing efficiency since the discriminator does not need to be executed at inference.

DI2IN may take the 3D imaging data ID as input, and outputs the probability maps that indicate how likely voxels belongs to the liver region. At least one block, in particular all blocks in DI2IN comprise 3D convolutional and bilinear upscaling layers.

In the encoder part of DI2IN, only the convolution layers are used in all blocks. In order to increase the receptive field of neurons and lower the GPU memory consumption, stride may be set as 2 at some layers and the size of feature maps may be reduced. Moreover, larger receptive field may cover more contextual information and help to preserve liver shape information in the prediction. The decoder of DI2IN may comprise convolutional and bilinear upscaling layers.

To enable end-to-end prediction and training, the upscaling layers may be implemented as bilinear interpolation to enlarge the activation maps. All convolutional kernels may be of 3 x 3 x 3. The upscaling factor in a decoder may be 2 for x; y; z dimension. The leaky rectified linear unit (Leaky ReLU) and batch normalization may be adopted in all convolutional layers for proper gradient back-propagation.

In order to further improve the performance of DI2IN, several mainstream technologies may be adopted. First, a feature layer concatenation may be used in DI2IN. Fast bridges may be built directly from the encoder layers to the decoder layers. The bridges may pass the information from the encoder forward and then may concatenate it with the decoder feature layers. The combined feature may be used as the input for the next convolution layer. Following the steps above to explicitly combine advanced and low-level features, DI2IN may benefit from local and global contextual information.

In a fourth method step 104 a second neural network is used to detect one or more suspected lesions of the segmented kidney representation. Therefore, the third neural network may be configured to classify the malignancy risk MR based on imaging data ID and non-imaging data NID, wherein the non-imaging data NID comprises at least histopathologic data.

The second neural network may be configured at least partly as a fully convolutional one-stage object detection (FCOS). This is preferably performed in a per-pixel prediction fashion, analogue to semantic segmentation. The FCOS may be anchor box free and/or proposal free. Thus, the FCOS may avoid any complicated computation related to anchor boxes such as calculating overlapping during training. Additionally, FCOS may avoid hyper-parameters related to anchor boxes, which may be sensitive to a final detection performance.

An example of a fully convolutional one-stage object decoder may be defined as follows: Let $F_i \in \mathbb{R}^{H \times W \times C}$ be the feature maps at layer I of a backbone CNN and s be the total stride until the layer. The ground-truth bounding boxes for the imaging data ID may be defined as {Bi}, where $B_i = \left( x_0^{(i)}, y_0^{(i)}, x_1^{(i)}, y_1^{(i)}, c^{(i)} \right) \in \mathbb{R}^4 \times \{1, 2 \ldots C\}$.

$\left( x_0^{(i)}, y_0^{(i)} \right)$ and $\left( x_1^{(i)}, y_1^{(i)} \right)$ may denote the coordinates of the left-top and right-bottom corners of the bounding box. $c^{(i)}$ may be the class that the object in the bounding box belongs to. C may be the number of classes. For each location (x, y) on the feature map $F_i$, one may map it back onto the input image as $\left( \left[ \frac{s}{2} \right] + xs, \left[ \frac{s}{2} \right] + ys \right)$, which is near the center of the receptive field of the location (x, y). The target bounding box may be directly regressed at the location.

Location (x, y) may be considered as a positive sample if it falls into any ground-truth box and the class label c* of the location may be the class label of the ground-truth box. Otherwise, it may be a negative sample and c* = 0 (background class). Furthermore, there may be a 4D real vector t* = (l*, t*, r*, b*) being the regression targets for the location. l*, t*, r* and b* may be distances from the location to the four sides of the bounding box. If a location falls into multiple bounding boxes, it may be considered as an ambiguous sample. The bounding box with minimal area may be chosen as its regression target.

If location (x, y) is associated to a bounding box $B_i$, the training regression targets for the location may be formulated as, l* = x-$x_0^{(i)}$, t* = y-$y_0^{(i)}$, r* = $x_1^{(i)}$-x and b* = $y_1^{(i)}$-y. The FCOS may leverage as many foreground samples as possible to train the regressor.

Corresponding to the training targets, the final layer of the networks may predict an 80D vector p of classification labels and a 4D vector t = (l, t, r, b) bounding box coordinates. In one example, C binary classifiers may be trained. In one example, at least four convolutional layers may be added after the feature maps of the backbone networks respectively for classification and regression branches. Since regression targets may be positive, exp(x) may be employed to map any real number to $(0,\infty)$ on the top of the regression branch.

A training loss function may be defined as follows:

$$L(\{p_{x,y}\}, \{t_{x,y}\}) = \frac{1}{N_{pos}} \sum_{x,y} L_{cls}(p_{x,y}, c^*_{x,y}) + \frac{\lambda}{N_{pos}} \sum_{x,y} 1_{\{c^*_{x,y}>0\}} L_{reg}(t_{x,y}, t^*_{x,y}),$$

where $L_{cls}$ may be focal loss and $L_{reg}$ may be the IOU loss. $N_{pos}$ may denote the number of positive samples and $\lambda$ being in 1 may be the balance weight for $L_{reg}$. The summation may be calculated over all locations on the feature maps $F_i$. $1_{\{c^*_{x,y}>0\}}$ may be the indicator function, being 1 if $c_i^* > 0$ and 0 otherwise.

Giving the imaging data ID, they may be forwarded through the network and may obtain the classification scores $p_{x,y}$ and the regression prediction $t_{x,y}$ for each location on the feature maps $F_i$. In one example, the required IOU scores for positive anchor boxes may be lowered.

In one example a single-layer branch may be added additionally, in parallel with a classification branch, in order to predict a "centerness" of a location. The centerness may depict the normalized distance from the location to the center of the object that the location is responsible for. Given the regression targets l*, t*, r* and b* for a location the centerness target may be defined as $centerness^* = \sqrt{\frac{\min(l^*,r^*)}{\max(l^*,r^*)} \times \frac{\min(t^*,b^*)}{\max(t^*,b^*)}}$ . The centerness may range from 0 to 1 and may be trained with binary cross entropy (BCE) loss. The final score may be computed by multiplying the predicted centerness with the corresponding classification score.

In a fifth method step 105 the detected suspected lesion may be classified with a malignancy risk MR using a third neural network, wherein the third neural network is a deep profiler. Therefore, the deep profiler may comprise an encoder for extracting imaging features. Furthermore, the encoder may be designed as convolutional neural network (CNN), in particular a three-dimensional CNN. Additionally, the deep profiler may comprise a decoder for estimating at least one malignancy risk MR indicator and/or a task-specific network for generating at least one image signature for classifying at least one malignancy risk MR.

In one example the imaging data ID may be quantified by intensity, geometry, texture and/or wavelet features. The geometry features may quantify the 2D or 3D shape characteristics of the kidney, the texture features may describe spatial distribution of voxel or pixel intensities, thereby quantifying a heterogeneity. Any intensity and texture features may be computed after applying wavelet transformations to the imaging data ID.

To find voxels of the imaging data ID that contribute the most toward the prediction, the derivative of the final partial likelihood loss with respect to the imaging data ID may be taken and evaluated.

In one example the imaging data ID and/or non-imaging data NID may comprise context data relating to data recording information. In addition, the context data may comprise at least one out of following: data recording device information, contrast configuration information, brightness configuration information, recording direction information, total recording time information, projection type information (e.g. CT: average intensity projection (AIP), maximum intensity projection (MIP) or Minimum intensity projection MinIP)), date and/or time of recording information.

In one example, the non-imaging data NID of the subject patient may comprise at least one out of following: age, weight, size, health condition information, gender, nutritional practice.

A sixth method step 106 may convert the imaging data ID from at least a partially 3D illustration of the anatomy of the subject patient to a 2D illustration of the anatomy of the subject patient. This method step may be performed at any time due to e.g. memory or computational limitations.

FIG. 2 is a schematic drawing depicting a system 200 for classifying a malignancy risk MR scoring of a kidney.

The system 200 comprises an interface 201 which is configured to provide imaging data ID of an anatomy of a subject patient, wherein the imaging data ID comprises at least partially a representation of a kidney of the subject patient. Therefore, imaging data ID will be forwarded, in particular together with non-imaging data, to the interface.

A first analyzing unit 202 of the system 200 is configured to use a first neural network to segment at least one region of the kidney representation which is based on the imaging data. A second analyzing unit 203 is also part of the system 200, wherein the second analyzing unit 203 is configured to use a second neural network to detect one or more suspected lesions of the segmented kidney representation. The system comprises additionally a deep profiler 204 which is configured to classify the detected suspected lesion with a malignancy risk MR.

In one example the deep profiler 204 is configured to classify the malignancy risk MR based on imaging data ID and non-imaging data NID, wherein the non-imaging data NID comprises at least histopathologic data.

Whenever a malignancy risk MR score has been determined by the system 200, the value may be stored in the system 200 or in a cloud-based memory storage system. In one example, at least parts of the system 200 may be deployed in a cloud-based system, which means that they do not have to be physically integrated in a box.

The system 200 may be configured that each input data and/or output data can be forwarded to each of its units, namely its interface 201, its first analyzing unit 202, its second analyzing unit 203 and/or its deep profiler 204. It is further denoted that the neural networks of the specific units may be designed analogue to the neural networks disclosed above relating to Fig. 1.

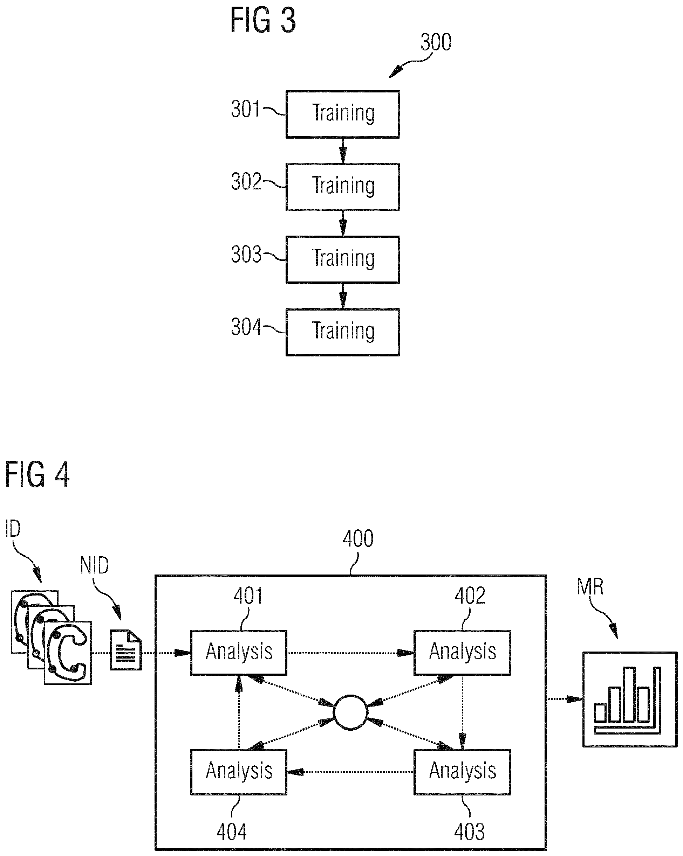

FIG. 3 is a schematic drawing depicting a computer-implemented method 300 for training a machine learning algorithm to classify a malignancy risk MR of a kidney.

Therefore, the method 300 comprises a first step 301 of training a first neural network with first training data including imaging data ID of an anatomy of at least one subject patient, wherein the imaging data ID comprises at least partially a representation of one or more kidneys. An adversarial network may be utilized for training of the first neural network to discriminate the output from ground truth.

In a second method step 302 a second neural network is trained with second training data including one or more detected lesions of one or more segmented kidney representations.

In a third method step 303 a third neural network is trained with third training data of one or more lesions classified with a malignancy risk MR. The third training data are preferably based on imaging data ID and non-imaging data NID, wherein the non-imaging data NID comprises at least histopathologic data. Furthermore, at least one ground-truth label of the deep profiler may be determined based on histopathologic data.

In a fourth method step 304 a fourth neural network is trained using deep reinforcement learning based on fourth training data related to detected anatomical landmarks, in particular landmarks relating to one or more kidney representations.

In one example at least two different training data fragments of the first, second third or fourth training data may be based on the same subject patient. Furthermore, the two different training data fragments may be indicated as training data of the same subject patient. Additionally, the non-imaging data NID may comprise at least one indicator which is configured to serve for patient follow-up diagnosis. FIG. 4 is a schematic drawing depicting a system 400 for training a machine learning algorithm to classify a malignancy risk MR of a kidney.

Therefore, the system 400 comprises a first analysis unit 401 which is configured to train a first neural network with first training data including imaging data ID of an anatomy of at least one subject patient, wherein the imaging data ID comprises at least partially a representation of one or more kidneys.

A second analysis unit 402 of the system 400 is configured to train a second neural network with second training data including one or more detected lesions of one or more segmented kidney representations. The system 400 further comprises a third analysis unit 403 which is configured to train a third neural network with third training data of one or more lesions classified with a malignancy risk MR.

The system may additionally comprise a fourth analysis unit 404 which is configured to train a fourth neural network with fourth training data related to detected anatomical landmarks, in particular landmarks relating to one or more kidney representations.

The system 400 may be configured that each input data and/or output data can be forwarded to each of its units, namely its first analysis unit 401, its second analysis unit 402, its third analysis unit 403 and/or its fourth analysis unit 404. It is further denoted that the neural networks of the specific units may be designed analogue to the neural networks disclosed above relating to Fig. 3.

**Claims**

1. A computer-implemented method (100) for classifying a malignancy risk (MR) of a kidney, in particular a human kidney, comprising following steps:

   - providing (101) imaging data (ID) of an anatomy of a subject patient, wherein the imaging data (ID) comprises at least partially a representation of a kidney of the subject patient;
   - using (103) a first neural network to segment at least one region of the kidney representation which is based on the imaging data (ID);
   - using (104) a second neural network to detect one or more suspected lesions of the segmented kidney representation; and
   - classifying (105) the detected suspected lesion with a malignancy risk (MR) using a third neural network,

   wherein the third neural network is a deep profiler.

2. The method (100) of claim 1, wherein the third neural network is configured to classify the malignancy risk (MR) based on imaging data (ID) and non-imaging data (NID), wherein the non-imaging data (NID) comprises at least histopathologic data.

3. The method (100) of claim 1 or 2, wherein the deep profiler comprises an encoder for extracting imaging features.

4. The method (100) of one of the preceding claims, wherein the encoder is designed as convolutional neural network, CNN, in particular a three-dimensional CNN.

5. The method (100) of at least one of the preceding claims, wherein the deep profiler comprises a decoder for estimating at least one malignancy risk (MR) indicator.

6. The method (100) of at least one of the preceding claims, wherein the deep profiler comprises a task-specific network for generating at least one image signature for classifying at least one malignancy risk (MR).

7. The method (100) of at least one of the preceding claims, comprising the additional method step:

   - using (102) a fourth neural network to detect anatomical landmarks based on the provided imaging data (ID), and preferably the fourth neural network is designed as convolutional neural network for image feature extraction and more preferably the fourth neural network additionally uses at least one universal non-linear function approximator.

8. The method (100) of one of the preceding claims, wherein the first neural network is designed as at least one of the following:

   - a convolutional encoder-decoder architecture.
   - a multi-level feature concatenation and deep supervision architecture.

9. The method (100) of at least one of the preceding claims, wherein the step of detecting one or more suspected lesions is based on a fully convolutional one-stage object detection.

10. The method (100) of at least one of the preceding claims, wherein the provided imaging data (ID) comprises at least one of the following:

    - is based on computer tomography and/or magnet resonance imaging,
    - comprises at least partially a 3D illustration of the anatomy of the subject patient.

11. The method (100) of at least one of the preceding claims, comprising the additional step:

    - converting (106) the imaging data (ID) from at least a partially 3D illustration of the anatomy of the subject patient to a 2D illustration of the anatomy of the subject patient.

12. A system (200) for classifying a malignancy risk (MR) scoring of a kidney, in particular a human kidney, comprising:

    - an interface (201) which is configured to provide imaging data (ID) of an anatomy of a subject patient, wherein the imaging data (ID) comprises at least partially a representation of a kidney of the subject patient;
    - a first analyzing unit (202) which is configured to use a first neural network to segment at least one region of the kidney representation which is based on the imaging data (ID);
    - a second analyzing unit (203) which is configured to use a second neural network to detect one or more suspected lesions of the segmented kidney representation; and
    - a deep profiler (204) which is configured to classify the detected suspected lesion with a malignancy risk.

13. The system (200) of claim 12, wherein the deep profiler (204) is configured to classify the malignancy risk (MR) based on imaging data (ID) and non-imaging data (NID), wherein the non-imaging data (NID) comprises at least histopathologic data.

14. A computer-implemented method (300) for training a machine learning algorithm to classify a malignancy risk (MR) of a kidney, in particular a human kidney, comprising following steps:

- training (301) a first neural network with first training data including imaging data (ID) of an anatomy of at least one subject patient, wherein the imaging data (ID) comprises at least partially a representation of one or more kidneys;
- training (302) a second neural network with second training data including one or more detected lesions of one or more segmented kidney representations; and
- training (303) a third neural network with third training data of one or more lesions classified with a malignancy risk.

15. A system (400) for training a machine learning algorithm to classify a malignancy risk (MR) of a kidney, in particular a human kidney, comprising:

- a first analysis unit (401) which is configured to train a first neural network with first training data including imaging data (ID) of an anatomy of at least one subject patient, wherein the imaging data (ID) comprises at least partially a representation of one or more kidneys;
- a second analysis unit (402) which is configured to train a second neural network with second training data including one or more detected lesions of one or more segmented kidney representations; and
- a third analysis unit (403) which is configured to train a third neural network with third training data of one or more lesions classified with a malignancy risk.

16. A computer program comprising instructions which, when the program is executed by a computer, cause the computer to carry out the method (100; 300) of at least one of the claims 1 to 11 and/or 14.

17. A machine-readable medium having stored thereon a set of instructions, which if performed by one or more processors, cause the one or more processors to carry out a method (100; 300) as mentioned in at least one of the claims 1 to 11 and/or 14.

**Patentansprüche**

1. Computerimplementiertes Verfahren (100) zum Klassifizieren eines Malignitätsrisikos (MR) einer Niere, insbesondere einer menschlichen Niere, umfassend die Schritte:

- Bereitstellen (101) von Bildgebungsdaten (ID) einer Anatomie eines Probanden/Patienten, wobei die Bildgebungsdaten (ID) wenigstens teilweise eine Darstellung einer Niere des Probanden/Patienten umfassen;
- Verwenden (103) eines ersten neuronalen Netzes, um wenigstens eine Region der Nierendarstellung zu segmentieren, die auf den Bildgebungsdaten (ID) basiert;
- Verwenden (104) eines zweiten neuronalen Netzes, um eine oder mehrere verdächtige Läsionen der segmentierten Nierendarstellung zu detektieren; und
- Klassifizieren (105) der detektierten verdächtigen Läsion mit einem Malignitätsrisiko (MR) unter Verwendung eines dritten neuronalen Netzes,

wobei das dritte neuronale Netz ein Deep Profiler ist.

2. Verfahren (100) nach Anspruch 1, wobei das dritte neuronale Netz dazu ausgelegt ist, das Malignitätsrisiko (MR) basierend auf Bildgebungsdaten (ID) und Nicht-Bildgebungsdaten (NID) zu klassifizieren, wobei die Nicht-Bildgebungsdaten (NID) wenigstens histopathologische Daten umfassen.

3. Verfahren (100) nach Anspruch 1 oder 2, wobei der Deep Profiler einen Codierer zum Extrahieren von Bildgebungsmerkmalen umfasst.

4. Verfahren (100) nach einem der vorstehenden Ansprüche, wobei der Codierer als faltendes neuronales Netz (Convolutional Neural Network, CNN), insbesondere als dreidimensionales CNN ausgestaltet ist.

5. Verfahren (100) nach wenigstens einem der vorstehenden Ansprüche, wobei der Deep Profiler einen Decodierer zum Schätzen wenigstens eines Malignitätsrisiko (MR)-Indikators umfasst.

6. Verfahren (100) nach wenigstens einem der vorstehenden Ansprüche, wobei der Deep Profiler ein aufgabenspezifisches Netz zum Generieren wenigstens einer Bildsignatur für das Klassifizieren wenigstens eines Malignitätsrisikos (MR) umfasst.

7. Verfahren (100) nach wenigstens einem der vorstehenden Ansprüche, umfassend den zusätzlichen Verfahrensschritt:

   - Verwenden (102) eines vierten neuronalen Netzes, um anatomische Landmarken basierend auf den bereitgestellten Bildgebungsdaten (ID) zu detektieren, und wobei das vierte neuronale Netz vorzugsweise als faltendes neuronales Netz zur Bildmerkmalsextraktion ausgestaltet ist und das vierte neuronale Netz, noch bevorzugter, zusätzlich wenigstens einen universellen nichtlinearen Funktionsapproximator verwendet.

8. Verfahren (100) nach einem der vorstehenden Ansprüche, wobei das erste neuronale Netz als wenigstens eines hiervon ausgestaltet ist:

   - eine faltende Codierer-Decodierer-Architektur.
   - eine Mehrebenen-Merkmalsverkettungs- und Tiefenüberwachungsarchitektur.

9. Verfahren (100) nach wenigstens einem der vorstehenden Ansprüche, wobei der Schritt des Detektierens von einer oder mehreren verdächtigen Läsionen auf einer vollständig faltenden einstufigen Objektdetektion basiert.

10. Verfahren (100) nach wenigstens einem der vorstehenden Ansprüche, wobei die bereitgestellten Bildgebungsdaten (ID) wenigstens eines der folgenden umfassen:

    - sie basieren einer auf Computertomographie- und/oder Magnetresonanzbildgebung,
    - sie umfassen wenigstens teilweise eine 3D-Darstellung der Anatomie des Probanden/Patienten.

11. Verfahren (100) nach wenigstens einem der vorstehenden Ansprüche, umfassend den zusätzlichen Schritt:

    - Umwandeln (106) der Bildgebungsdaten (ID) von einer wenigstens teilweisen 3D-Darstellung der Anatomie des Probanden/Patienten in eine 2D-Darstellung der Anatomie des Probanden/Patienten.

12. System (200) zum Klassifizieren einer Malignitätsrisiko (MR)-Bewertung einer Niere, insbesondere einer menschlichen Niere, umfassend:

    - eine Schnittstelle (201), die dazu ausgelegt ist, Bildgebungsdaten (ID) einer Anatomie eines Probanden/-Patienten bereitzustellen, wobei die Bildgebungsdaten (ID) wenigstens teilweise eine Darstellung einer Niere des Probanden/Patienten umfassen;
    - eine erste Analyseeinheit (202), die dazu ausgelegt ist, ein erstes neuronales Netz zu verwenden, um wenigstens eine Region der Nierendarstellung zu segmentieren, die auf den Bildgebungsdaten (ID) basiert;
    - eine zweite Analyseeinheit (203), die dazu ausgelegt ist, ein zweites neuronales Netz zu verwenden, um eine oder mehrere verdächtige Läsionen der segmentierten Nierendarstellung zu detektieren; und
    - einen Deep Profiler (204), der dazu ausgelegt ist, die detektierte verdächtige Läsion mit einem Malignitätsrisiko zu klassifizieren.

13. System (200) nach Anspruch 12, wobei der Deep Profiler (204) dazu ausgelegt ist, das Malignitätsrisiko (MR) basierend auf Bildgebungsdaten (ID) und Nicht-Bildgebungsdaten (NID) zu klassifizieren, wobei die Nicht-Bildgebungsdaten (NID) wenigstens histopathologische Daten umfassen.

14. Computerimplementiertes Verfahren (300) zum Trainieren eines Maschinenlernalgorithmus zum Klassifizieren eines Malignitätsrisikos (MR) einer Niere, insbesondere einer menschlichen Niere, umfassend die folgenden Schritte:

    - Trainieren (301) eines ersten neuronalen Netzes mit ersten Trainingsdaten, beinhaltend Bildgebungsdaten (ID) einer Anatomie wenigstens eines Probanden/Patienten, wobei die Bildgebungsdaten (ID) wenigstens teilweise eine Darstellung einer oder mehrerer Nieren umfassen;
    - Trainieren (302) eines zweiten neuronalen Netzes mit zweiten Trainingsdaten, beinhaltend eine oder mehrere detektierte Läsionen einer oder mehrerer segmentierter Nierendarstellungen; und
    - Trainieren (303) eines dritten neuronalen Netzes mit dritten Trainingsdaten einer oder mehrerer Läsionen, die mit einem Malignitätsrisiko klassifiziert sind.

**15.** System (400) zum Trainieren eines Maschinenlernalgorithmus zum Klassifizieren eines Malignitätsrisikos (MR) einer Niere, insbesondere einer menschlichen Niere, umfassend:

- eine erste Analyseinheit (401), die dazu ausgelegt ist, ein erstes neuronales Netz mit ersten Trainingsdaten zu trainieren, beinhaltend Bildgebungsdaten (ID) einer Anatomie wenigstens eines Probanden/Patienten, wobei die Bildgebungsdaten (ID) wenigstens teilweise eine Darstellung einer oder mehrerer Nieren umfassen;
- eine zweite Analyseinheit (402), die dazu ausgelegt ist, ein zweites neuronales Netz mit zweiten Trainingsdaten zu trainieren, beinhaltend eine oder mehrere detektierte Läsionen einer oder mehrerer segmentierter Nierendarstellungen; und
- eine dritte Analyseeinheit (403), die dazu ausgelegt ist, ein drittes neuronales Netz mit dritten Trainingsdaten einer oder mehrerer Läsionen zu trainieren, die mit einem Malignitätsrisiko klassifiziert sind.

**16.** Computerprogramm, umfassend Anweisungen, die, wenn das Programm von einem Computer ausgeführt wird, den Computer veranlassen, das Verfahren (100; 300) nach wenigstens einem der Ansprüche 1 bis 11 und/oder 14 auszuführen.

**17.** Maschinenlesbares Medium, auf dem ein Satz von Anweisungen gespeichert ist, die, falls sie von einem oder mehreren Prozessoren durchgeführt werden, die ein oder mehreren Prozessoren veranlassen, ein Verfahren (100; 300) wie in wenigstens einem der Ansprüche 1 bis 11 und/oder 14 erwähnt auszuführen.

## Revendications

**1.** Procédé informatique (100) permettant de classer un risque de malignité (MR) d'un rein, en particulier d'un rein humain, comprenant les étapes suivantes :

- la fourniture (101) de données d'imagerie (ID) d'une anatomie d'un patient sujet, les données d'imagerie (ID) comprenant au moins partiellement une représentation d'un rein du patient sujet ;
- l'utilisation (103) d'un premier réseau neuronal pour segmenter au moins une région de la représentation du rein qui est basée sur les données d'imagerie (ID) ;
- l'utilisation (104) d'un deuxième réseau neuronal pour détecter une ou plusieurs lésions suspectées de la représentation du rein segmentée ; et
- le classement (105) de la lésion suspectée à risque de malignité (RM) détectée à l'aide d'un troisième réseau neuronal, le troisième réseau neuronal étant un profileur profond.

**2.** Procédé (100) selon la revendication 1, le troisième réseau neuronal étant configuré pour classer le risque de malignité (MR) sur la base de données d'imagerie (ID) et de données de non-imagerie (NID), les données de non-imagerie (NID) comprenant au moins des données histopathologiques.

**3.** Procédé (100) selon la revendication 1 ou 2, le profileur profond comprenant un codeur pour extraire des caractéristiques d'imagerie.

**4.** Procédé (100) selon l'une quelconque des revendications précédentes, le codeur étant réalisé sous la forme d'un réseau neuronal convolutif, CNN, en particulier d'un CNN tridimensionnel.

**5.** Procédé (100) selon au moins l'une des revendications précédentes, le profileur profond comprenant un décodeur pour estimer au moins un indicateur de risque de malignité (MR).

**6.** Procédé (100) selon au moins l'une des revendications précédentes, le profileur profond comprenant un réseau de tâche spécifique pour générer au moins une signature d'image pour classer au moins un risque de malignité (MR).

**7.** Procédé (100) selon au moins l'une des revendications précédentes, comprenant l'étape de procédé supplémentaire :

- l'utilisation (102) d'un quatrième réseau neuronal pour détecter des repères anatomiques sur la base des données d'imagerie (ID) fournies, et de préférence le quatrième réseau neuronal étant conçu comme un réseau neuronal convolutif pour l'extraction de caractéristiques d'image et, de préférence, le quatrième réseau neuronal utilisant en outre au moins un approximateur de fonction non linéaire universel.

**8.** Procédé (100) selon l'une des revendications précédentes, le premier réseau neuronal étant conçu comme au moins l'un des suivants :

- une architecture codeur-décodeur convolutionnelle.
- une concaténation de fonctionnalités multi-niveaux et une architecture de supervision approfondie.

**9.** Procédé (100) selon au moins l'une des revendications précédentes, l'étape de détection d'une ou plusieurs lésions suspectées étant basée sur une détection d'objet entièrement convolutionnelle en une étape.

**10.** Procédé (100) selon au moins l'une des revendications précédentes, les données d'imagerie fournies (ID) comprenant au moins l'un des cas suivants :

- étant basées sur la tomographie assisté par ordinateur et/ou l'imagerie par résonance magnétique,
- comprenant au moins partiellement une illustration 3D de l'anatomie du patient sujet.

**11.** Procédé (100) selon au moins l'une des revendications précédentes, comprenant l'étape supplémentaire :

- la conversion (106) des données d'imagerie (ID) d'au moins une illustration 3D partielle de l'anatomie du patient sujet en une illustration 2D de l'anatomie du patient sujet..

**12.** Système (200) de classement d'un score de risque de malignité (MR) d'un rein, en particulier d'un rein humain, comprenant :

- une interface (201) qui est configurée pour fournir des données d'imagerie (ID) d'une anatomie d'un patient sujet, les données d'imagerie (ID) comprenant au moins partiellement une représentation d'un rein du patient sujet ;
- une première unité d'analyse (202) qui est configurée pour utiliser un premier réseau neuronal pour segmenter au moins une région de la représentation du rein qui est basée sur les données d'imagerie (ID) ;
- une deuxième unité d'analyse (203) qui est configurée pour utiliser un deuxième réseau neuronal pour détecter une ou plusieurs lésions suspectées de la représentation du rein segmentée ; et
- un profileur profond (204) qui est configuré pour classer la lésion suspectée à risque de malignité détectée.

**13.** Système (200) selon la revendication 12, le profileur profond (204) étant configuré pour classer le risque de malignité (MR) sur la base de données d'imagerie (ID) et de données de non-imagerie (NID), les données de non-imagerie (NID) comprenant au moins des données histopathologiques.

**14.** Procédé informatique (300) permettant d'entraîner un algorithme d'apprentissage automatique afin de classer un risque de malignité (MR) d'un rein, en particulier d'un rein humain, comprenant les étapes suivantes :

- l'entraînement (301) d'un premier réseau neuronal avec des premières données d'entraînement comprenant des données d'imagerie (ID) d'une anatomie d'au moins un patient sujet, les données d'imagerie (ID) comprenant au moins partiellement une représentation d'un ou plusieurs reins ;
- l'entraînement (302) d'un deuxième réseau neuronal avec des deuxièmes données d'entraînement comprenant une ou plusieurs lésions détectées d'une ou plusieurs représentations du rein segmentées ; et
- l'entraînement (303) d'un troisième réseau neuronal avec des troisièmes données d'entraînement d'une ou plusieurs lésions classées avec un risque de malignité.

**15.** Système (400) d'entraînement d'un algorithme d'apprentissage automatique pour classer un risque de malignité (MR) d'un rein, en particulier d'un rein humain, comprenant :

- une première unité d'analyse (401) qui est configurée pour entraîner un premier réseau neuronal avec des premières données d'entraînement comprenant des données d'imagerie (ID) d'une anatomie d'au moins un patient sujet, les données d'imagerie (ID) comprenant au moins partiellement une représentation d'un ou plusieurs reins ;
- une deuxième unité d'analyse (402) qui est configurée pour entraîner un deuxième réseau neuronal avec des deuxièmes données d'entraînement comprenant une ou plusieurs lésions détectées d'une ou plusieurs représentations du rein segmentées ; et
- une troisième unité d'analyse (403) qui est configurée pour entraîner un troisième réseau neuronal avec des troisièmes données d'entraînement d'une ou plusieurs lésions classées avec un risque de malignité.

16. Programme informatique comprenant des instructions qui, lorsque le programme est exécuté par un ordinateur, amènent l'ordinateur à mettre en œuvre le procédé (100 ; 300) selon au moins l'une des revendications 1 à 11 et/ou 14.

17. Support lisible par machine sur lequel est stocké un ensemble d'instructions qui, si elles sont réalisées par un ou plusieurs processeurs, amènent le ou les processeurs à mettre en œuvre un procédé (100 ; 300) selon au moins l'une des revendications 1 à 11 et/ou 14.

# FIG 1

100

101 — Provide imaging data

102 — Detect anatomical landmarks

103 — Segment at least one region

104 — Detect suspected lesions

105 — Classify malignancy risk

106

Conversion

# FIG 2

ID

NID

200

201

Interface

202

Analyze

203

Analyze

204

Deep profiler

MR

FIG 3

300

301 — Training

302 — Training

303 — Training

304 — Training

FIG 4

ID

NID

400

401 Analysis

402 Analysis

MR

404 Analysis

403 Analysis

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Deep Learning in Computer-Aided Diagnosis and Treatment of Tumors: A Survey. **DAN ZHAO et al.** ARXIV.ORG. Cornell University Library, 02 November 2020, 1-17

- Applications of deep learning for the analysis of medical data. **HYUN-JONG JANG et al.** Archives of Pharmacal Research. Natl. Fisheries University, 28 May 2019, vol. 42, 492-504